# EUROPEAN PATENT APPLICATION

(11) **EP 0 691 279 A2**
(43) Date of publication of application: **10.01.1996**
(21) Application number: 95304680.2
(22) Date of filing: 04.07.1995
(51) Int. Cl.: B65D 47/26, B65D 47/08, A61M 5/32

(54) **Container for medical waste**

(30) Priority: 04.07.1994 GB 9413451
(71) Applicant: DRG PLASTICS LIMITED, Hereford HR2 6LD (GB)
(72) Inventor: Carrington, Geoffrey Robert, Hereford HR2 6DT (GB)
(74) Representative: Stuart, Ian Alexander

(57) **Abstract**

A container has a closure assembly (10) with an apertured wall (12,24) and an obstructor member (50) mounted to the wall so as to be movable (e.g. by rotation) from an open configuration to a closed configuration in which it obstructs the aperture (24). It may then be moved (e.g. axially) towards the aperture to a sealing configuration in which a sealing gasket (64) seals around the aperture.

## Description

The present invention relates to a container and to a closure therefor. It particularly relates to a container and closure for use in the disposal of medical waste.

Our earlier European specification, EP-A-0,367,422 discloses a closure assembly for a container, particularly a "sharps bin". It provides a container lid having an access aperture and an obstructor member which is slidable between an obstructing configuration and an open configuration. In the obstructing configuration, it closes the access aperture. It includes a locking piece including a slidable pin. When the obstructor member is in the obstructing configuration, the pin can be depressed so that it projects beneath the obstructor member and engages behind a detent formation of the lid, thus preventing the obstructor member from being slid out of the obstructing configuration.

In one aspect, the present invention provides a closure assembly for a container comprising a wall member having an access aperture, and an obstructor member mounted on the wall member so as to be displaceable between an open configuration in which the access aperture is accessible, and an obstructing configuration; wherein the obstructor member has a portion which is in register with the access aperture in the obstructing configuration; at least that portion of the obstructor member being displaceable towards the aperture in the obstructing configuration, and comprising sealing means for sealing around the access aperture.

Preferably the displacement of the obstructor member between the open and obstructing configurations involves motion generally in the plane of the wall member, e.g. rotary motion; and the displacement towards the aperture to effect sealing around it involves displacement having at least a component into the plane of the wall member.

There may be just a portion of the obstructor member that is displaceable towards the aperture for sealing, e.g. a hinged flap. Preferably, however, the entire obstructor member is displaced. Thus the obstructor member may be rotatably mounted on the wall member so as to be rotatable between the open and obstructing configurations. The sealing displacement may then involve axial displacement of the member. Preferably the axial displacement is possible only when the obstructor member is in the obstructing configuration, e.g. because then a projection on the obstructor member and a recess or opening on the wall member (or vice versa) are in register. Preferably the arrangement is such that the obstructor member is locked in the sealing configuration and is resistant to withdrawal. Thus the axial movement may involve an abutting portion moving beyond a projection which must be temporarily deformed to permit this. The shaping of the abutment and the projection may be mutually adapted so that motion in the reverse direction is resisted. Thus one component may be chamfered to facilitate the sealing motion, whereas its profile at the other side is abrupt, so as to resist the contrary motion.

The sealing means may be a gasket of sealing material which entirely covers, and extends laterally beyond, the access aperture. Alternatively it may be an annular seal in the region of the periphery of the access aperture (when the obstructor member is in the obstructing configuration). The gasket may be a closed-cell foam material, e.g. of PVC or polyurethane.

The closure assembly could be integral with a container. But more normally, it will be a separate component (generally a lid) for connection thereto. In another aspect, the invention provides a combination of a lid and a container, each having a respective rim portion by which they are mutually engageable, there being complementary engagement formations including sealing means such that their engagement produces a liquid-tight seal. Generally they will be snap-engageable, one component having a flange which snap-engages behind a flange of the other component. It can be arranged that this snap-engagement causes resilient compression of a seal element. Preferred embodiments incorporate both aspects.

Some embodiments of the invention will now be described in more detail with reference to the accompanying drawings in which:
Fig. 1 is a plan view of part of a first embodiment, namely a lid without an obstructor member;
Fig. 2 is a perspective view from beneath of an obstructor member for use with the lid of Fig. 1;
Figs. 3 and 4 are sectional views of the rim region of the obstructor member taken at III-III and IV-IV of Fig. 2 respectively.
Figs. 5 and 6 are a pair of perspective views from above of a second embodiment of the invention, partly cut away;
Fig. 7 is a perspective view from above showing part of a third embodiment; and
Fig. 8 is a vertical section through the region of connection of a lid and a container.

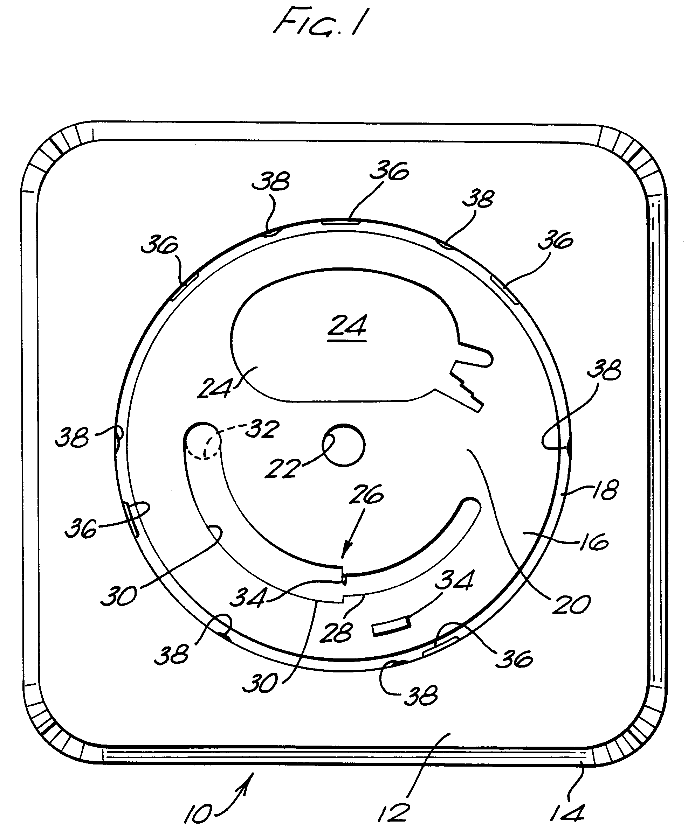

Fig. 1 shows a lid member 10 having a main wall 12 which is generally square with rounded corners. A peripheral wall 14 extends downwardly and outwardly from the periphery. The main wall 12 has a recessed circular central region 16, delimited by an outer annular channel 18. Within this there is an inner wall 20 penetrated by a central circular socket 22 and a large access opening 24. It has a recess defining a coaxial arcuate channel 26 having a narrow portion 28 and a wider portion 30 which, in some embodiments, terminates in a deeper cavity 32. At the junction between the narrow and wide portions 28,30, there is a radial nib 34 that further narrows the mouth of the narrow portion 28. The inner wall 20 also has a small recess 34, radially outwardly of the arcuate channel.

The annular channel 18 is defined by an outer wall having radial projections. These form two sets differing in axial position. There is a lower set 36, in this example numbering five relatively large projections; and there is an upper set 38, in this example numbering six smaller projections. The upper surfaces of the lower projections 36 define a level which is spaced beneath the level defined by the lower surfaces of the upper projections. The lower projections are spaced above the floor of the annular channel 18.

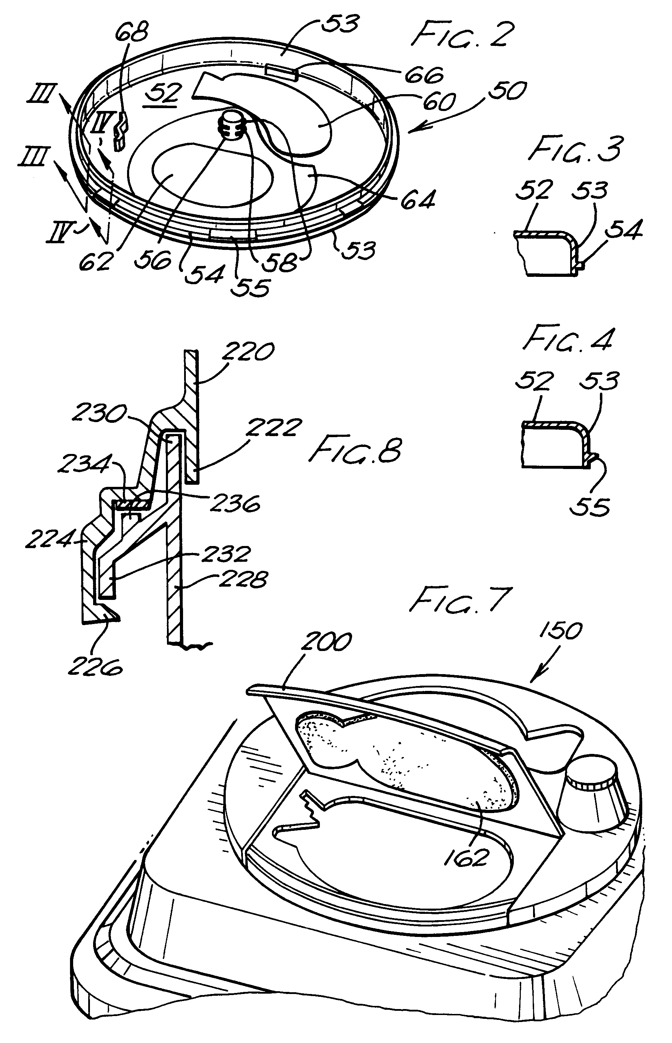

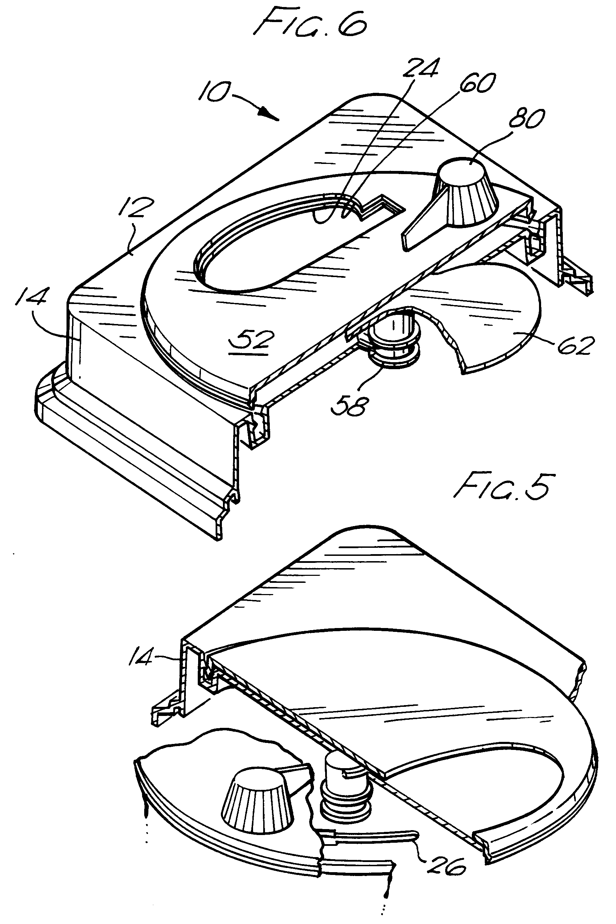

The obstructor member 50 shown in Figs. 2 to 4 is a circular disc having a main circular, planar wall 52 with a depending peripheral rim 53. At a lower region, this has an outwardly extending flange 54. This is generally of approximately rectangular section, as shown in Fig. 3. However, in a plurality of regions corresponding in number and angular distribution to the lower projections 36 in the annular channel 18 of the lid, the flange has a form shown in Fig. 4, with a lower surface 55 which is chamfered, or otherwise made more gradual.

The undersurface of the circular wall 52 has a central tubular spigot 56 with a spaced pair of interrupted circumferential ribs 58. There is a large access opening 60 capable of overlying the access aperture 24 of the lid. A region of the member 50 is recessed, so as to provide a large projection 62 on the lower face. This is embraced by a gasket member 64, which also surrounds the spigot 56. The gasket is a flat pad of resilient closed-cell PVC foam. The projection 62 is somewhat smaller than the access aperture 24 of the lid.

The underside of the member 50 also has a small, radially outer projection 66 and an approximately W-shaped projection 68. On the top side of the member 50, the recess corresponding to the projection 62 serves as a handgrip. This may be aided by one or more ribs extending across it.

The lid and obstructor member are dimensioned so that the member 50 can be placed on the lid, with the peripheral wall of the member received in the annular channel 18, and the spigot 56 engaged in the central aperture 22. The member is pushed down so that the outer flange 54 snap-engages under the upper projections 38, and rests on the upper surfaces of the lower projections 36; and the edge of the central aperture engages between the projections 58. The W-shaped projection 68 extends into the arcuate channel 26, whose extent thus delimits the rotation of the member. When it is fully rotated anti-clockwise, its access opening 60 is in register with the access aperture 24 of the lid. As it is rotated clockwise, there is a perceptible click as the W-shaped projection passes the nib 34. When the member 50 is rotated clockwise as far as it can go, the small outer projection 66 is then in register with the recess 34 of the lid. To effect locking of the obstructor member, it can then be pushed down. The spigot 56 is received more deeply, so that engagement is above the upper projection 58. Furthermore, the peripheral flange 54 is pushed past the lower projections 36, and engages beneath them. This pushing past is made possible by the fact that, in the fully clockwise configuration, it is the chamfered regions of the flange which overlie the lower projections 36. Thus the member 50 is locked down, held against rotation by the engagement of the small projection 66 in the recess 34, and the gasket 64 seals around the access opening 24 and also the central opening 22 of the lid. The sealing can be reliably fluid-tight.

Figs. 5 and 6 show a similar embodiment, and corresponding elements bear the same reference numbers. This embodiment also has features disclosed in our earlier application EP-A-0,367,422. Thus, instead of having a fixed W-shaped projection 68 that engages in the arcuate channel 26 of the lid, the obstructor member has a pin assembly 80 as disclosed in that earlier application. As shown in Fig. 1, the arcuate channel terminates in a deeper recess 32. When the pin reaches this, it can be depressed to lock therein. The pin assembly also provides a handgrip. There is no recess 62 in the obstructor member. The gasket 64 may be a complete pad, rather than an annulus. It may be adhered to the obstructor element.

Fig. 7 shows another variant in which sealing about the access aperture is effected by moving only a part of the obstructor member 150. The gasket 162 is carried on a hinged flap 200 which is a part of the obstructor member, having formations that snap-engage behind projections in the annular channel 18 of the lid, when it is pushed down. In this example, the hinged flap is an integrally-formed part of the member 150, with the hinge being provided by a line of weakness. But it could be a separate element, with a hinge connection.

The engagement of the lid on a container generally involves a rim portion of one component being received in a channel of the other component with some snap-engagement. Fig. 8 shows a variant of this that provides sealing. Thus one component 220 (which may be the lid) has a channel defined between an inner portion 222 and an outer portion 224, which is of greater extent and terminates in an inwardly extending rib 226. The other component 228 has a rim portion 230 that extends into the channel between the portions 222,224 of the other component. It also has an outwardly extending flange 232 which is inwardly resiliently deformable, and is dimensioned to snap-engage behind the rib 226. In this modified version, the outer flange 224 of the first component and the flange 232 of the second component are modified to provide a seat for a gasket element and a sealing face. In this example, the flange 224 of the first component is stepped to provide a surface for a sealing element 234, and the flange 232 of the second component has an upstanding element that provides a sealing surface 236.

## Claims

1. **1.** A closure assembly (10) for a container comprising a wall member (12) having an access aperture (24), and an obstructor member (50) mounted on the wall member (12) so as to be displaceable between an open configuration in which the access aperture (24) is accessible, and an obstructing configuration; wherein the obstructor member (50) has a portion which is in register with the access aperture (24) in the obstructing configuration; characterised in that at least said portion of the obstructor member (50) is displaceable towards the aperture (24) in the obstructing configuration to provide a sealing configuration, there being sealing means (63) for sealing around the access aperture (24).

2. **2.** An assembly according to claim 1 wherein the displacement of the obstructor member (50) between the open and obstructing configurations involves motion generally in the plane of the wall member (12), and the displacement towards the aperture (24) to effect sealing around it involves displacement having at least a component into the plane of the wall member (12).

3. **3.** An assembly according to claim 1 or claim 2 wherein the displacement of the obstructor member (50) between the open and obstructing configurations involves rotary motion generally in the plane of the wall member (12).

4. **4.** An assembly according to claim 3 wherein the obstructor member (50) is rotatably mounted on the wall member (12) so as to be rotatable between the open and obstructing configurations, and displaceable axially to the sealing configuration.

5. **5.** An assembly according to claim 4 wherein the axial displacement is possible only when the obstructor member (50) is in the obstructing configuration.

6. **6.** An assembly according to claim 4 wherein there are a projection (66) on the obstructor member (50) and a recess or opening (34) on the wall member (12) (or vice versa) which are in register in the obstructing configuration.

7. **7.** An assembly according to any of claims 1-3 wherein the obstructor member includes a hinged flap (200) which moves hingedly to the sealing configuration.

8. **8** An assembly according to any preceding claim wherein the arrangement is such that the obstructor member (50) is locked in the sealing configuration and is resistant to withdrawal.

9. **9.** An assembly according to any preceding claim wherein the sealing means is a gasket (64) of sealing material which entirely covers, and extends laterally beyond, the access aperture.

10. **10.** An assembly according to any of claims 1-8 wherein the sealing means (64) is an annular seal which is located in the region of the periphery of the access aperture (24) (when the obstructor member is in the obstructing configuration).

11. **11.** An assembly according to any preceding claim wherein the sealing means (64) comprises a closed-cell foam material.

12. **12.** A combination of a lid (220) and a container (228), each having a respective rim portion (222,224;230,232) by which they are mutually engageable, there being complementary engagement formations (232,226) including sealing means (234) such that their engagement produces a liquid-tight seal.

13. **13.** A combination according to claim 12 wherein the lid incorporates a closure assembly according to any of claims 1-11 .
